# EUROPÄISCHE PATENTSCHRIFT

(11) **EP 2 836 247 B1**
(45) Veröffentlichungstag und Bekanntmachung des Hinweises auf die Patenterteilung: **23.12.2015**
(21) Anmeldenummer: 14718080.6
(22) Anmeldetag: 16.04.2014
(51) Int. Cl.: A61M 1/36, A61M 39/22

(54) **REZIRKULATIONSVORRICHTUNG EINES EXTRAKORPORALEN BLUTBEHANDLUNGSGERÄTS**
RECIRCULATION DEVICE OF AN EXTRACORPOREAL BLOOD TREATMENT APPARATUS
DISPOSITIF DE RECIRCULATION D'UN APPAREIL DE TRAITEMENT SANGUIN EXTRACORPOREL

(30) Priorität: 19.04.2013 DE 102013103986
(43) Veröffentlichungstag der Anmeldung: 18.02.2015
(73) Patentinhaber: B. Braun Avitum AG, 34212 Melsungen (DE)
(72) Erfinder: HECTOR, Rainer, 49082 Osnabrück (DE); HUNDERTMARK, Charleen, 34119 Kassel (DE); HIERONYMI, Judith, 34281 Gudensberg (DE); SUSCA, Michele, 69221 Dossenheim (DE); MIHAILESCU, Anne-Marie, 34212 Melsungen (DE); FREITAG, Claudia, 34281 Gudensberg (DE)
(74) Vertreter: Winter, Brandl, Fürniss, Hübner, Röss, Kaiser, Polte - Partnerschaft mbB
(86) Internationale Anmeldenummer: PCT/EP2014/057749
(87) Internationale Veröffentlichungsnummer: WO 2014/170382

(56) Entgegenhaltungen:
- US-A- 4 819 653
- US-A- 5 334 315
- US-A- 5 540 653

## Beschreibung

Die vorliegende Erfindung betrifft die Rezirkulationsvorrichtung eines extrakorporalen Blutbehandlungsgeräts insbesondere einer Dialysemaschine gemäß dem Oberbegriff des Anspruchs 1.

### Hintergrund der Erfindung

Das hydraulische System eines Blutbehandlungsgeräts, beispielsweise einer Dialysemaschine muss vor Anschluss an einen Patienten mit einer Flüssigkeit beispielsweise einer NaCl - Lösung gefüllt werden, derart, dass Lufteinschlüsse in dem System entlüftet werden, die für einen, an das System flüssigkeits - angeschlossenen Patienten gefährlich sein würden. Des Weiteren kann das hydraulische System mit der eingefüllten Flüssigkeit über einen bestimmten Zeitraum durchgespült werden, um im System möglicherweise abgelagerte Schadstoffe, Schmutzpartikel, etc. auszufiltern/auszuschwemmen, bevor das System am Patienten angelegt wird. Diese beiden Vorgänge werden bei einem extrakorporalen Blutbehandlungsgerät im Rahmen eines Befüll - Rezirkulations - Zyklus durchgeführt.

### Stand der Technik

Aus US 5,540,653 ist eine Vorrichtung zur Befüllung eines extrakorporalen Blutbehandlungsgeräts bekannt, bei der ein Flüssigkeitsbehälter über eine 3-Wege-Weiche mit einem venösen Leitungsabschnitt verbunden ist.

Im Stand der Technik existieren Flüssigkeitsbehälter vorzugsweise in Form von Kunststoffbeuteln, die speziell für extrakorporale Blutbehandlungsgeräte dieser einschlägigen Gattung konstruiert sind, um die u. a. wie vorstehend definierten Gerätefunktionen zu ermöglichen. Solche Flüssigkeitsbehälter werden auch von der Anmelderin der vorliegenden Anmeldung hergestellt und vertrieben.

Ein solcher Flüssigkeitsbehälter hat in der Regel eine Flüssigkeitsaufnahmekammer und zwei vorzugsweise verschließbare Flüssigkeitsanschlüsse. An einen ersten der zwei Anschlüsse ist ein arterieller Leitungsabschnitt und an den zweiten Anschluss ein venöser Leitungsabschnitt des hydraulischen Systems (Fluidsystems) des extrakorporalen Blutbehandlungsgeräts anschließbar. Der Flüssigkeitsbeutel sowie die beiden Leitungsabschnitte bilden zusammen eine Rezirkulationsvorrichtung des extrakorporalen Blutbehandlungsgeräts

Für den Fluidsystem - Befüllvorgang wird zunächst der arterielle Leitungsabschnitt an den ersten Flüssigkeitsanschluss des Beutels angeschlossen und nach Öffnen des ersten Flüssigkeitsanschlusses das hydraulische System befüllt. Dabei verbleibt der venöse Leitungsabschnitt des Systems zunächst zur Atmosphäre hin offen (oder ist an einen Ablauf angeschlossen), sodass im System befindliche Luft zur Atmosphäre hin entweichen/entlüftet werden kann. Sobald der Befüllvorgang abgeschlossen ist, wird der venöse Leitungsabschnitt an den zweiten Flüssigkeitsanschluss des Beutels angeschlossen, um dann die im hydraulische System des extrakorporalen Blutbehandlungsgeräts befindliche Flüssigkeit für eine bestimmte Zeitspanne oder um ein bestimmtes Strömungsvolumen über die Beutelkammer zu rezirkulieren.

Bei diesem Rezirkulationsvorgang durchströmt die Flüssigkeit systeminterne Filtereinrichtungen, in denen möglicherweise vorhandene Verunreinigungen und/oder restliche Lufteinschlüsse mit der Flüssigkeit entfernt/ausgefiltert werden, welche beispielsweise bei der Herstellung der Systemkomponenten wie Schläuche, Ventile, etc. entstehen und sich im System ablagern können. Schließlich wird der venöse Leitungsabschnitt des hydraulischen Systems wieder vom zweiten Flüssigkeitsanschluss des Fluidbeutels abgeklemmt und die im hydraulischen System befindliche Flüssigkeit unter ständiger Zufuhr von Flüssigkeit aus dem Behälter nochmals ausgespült.

Mit Beendigung des Rezirkulationsvorgangs ist der eine Patientenbehandlung vorbereitende Befüll-/Rezirkulationszyklus abgeschlossen, sodass die beiden Leitungsabschnitte (venös und arteriell) vom Flüssigkeitsbeutel abgetrennt und an den Patienten für eine Behandlung angelegt werden können.

Aus der vorstehenden Beschreibung des Befüll-/Rezirkulationszyklus eines aus dem Stand der Technik bekannten hydraulischen Systems/Flüssigkeitsleitungssystems eines extrakorporalen Blutbehandlungsgeräts (Dialysemaschine) wird ersichtlich, dass für die Befüll- und Rezirkulationsvorgänge der Fluidbeutel im Systemkreis verbleibt, d. h. die im System befindliche Flüssigkeit durch den Fluidbeutel bzw. durch dessen Fluidkammer zirkuliert wird und damit die im Fluidbeutel bevorratete Flüssigkeit regelmäßig kontaminiert. Dies hat zur Folge, dass mit jeder neuen Behandlungsvorbereitung des extrakorporalen Blutbehandlungsgeräts ein neuer Fluidbeutel mit frischer, unkontaminierter Flüssigkeit für den folgenden Befüll-/Rezirkulationszyklus eingesetzt werden muss, wohingegen der Fluidbeutel für den zuvor ausgeführten Befüll-/Rezirkulationszyklus entsorgt wird, unabhängig von dessen Restfüllstand. Es liegt auf der Hand, dass bei dieser Vorgehensweise im Fall einer hohen Behandlungszahl ein mengenmäßig großer Teil an Flüssigkeit verschwendet wird, da der Flüssigkeitsinhalt eines Flüssigkeitsbeutels nur für einen Befüll-/Rezirkulationszyklus (unvollständig) verwendbar ist.

Angesichts dieser Problematik ist es die Aufgabe der vorliegenden Erfindung, eine Rezirkulationsvorrichtung eines extrakorporalen Blutbehandlungsgeräts bereit zu stellen, die gegenüber dem Stand der Technik wirtschaftlicher und damit kostengünstiger betrieben werden kann.

Diese Aufgabe wird durch eine Rezirkulationsvorrichtung eines extrakorporalen Blutbehandlungsgeräts mit den Merkmalen des Anspruchs 1 gelöst. Vorteilhafte Ausgestaltungen der Erfindung sind Gegenstand der Unteransprüche.

Der Kern der vorliegenden Erfindung besteht darin, die gattungsgemäße Rezirkulationsvorrichtung eines extrakorporalen Blutbehandlungsgeräts mit einer 3 - Wege - Weiche, beispielsweise einem Y- oder T- Stück oder einem 3 - Wege - Hahn auszustatten, wobei die Wege wahlweise absperrbar sind (beispielsweise über Schlauchklemmen an den Wegen oder über den Hahn), um so die Weiche vollständig zu verschließen und/oder die Wege wahlweise miteinander fluid zu koppeln bzw. ein Fluidströmung zumindest zwischen zwei ausgewählten Wegen zuzulassen. Erfindungsgemäß ist die 3 - Wege - Weiche, vorzugsweise der 3 - Wege - Hahn unmittelbar diesen einem vorzugsweise universellen medizinischen Flüssigkeitsbehälter nachgeordnet. Im Konkreten wird ein erster Anschluss der 3 - Wege - Weiche (des 3 - Wege - Hahns) vorzugsweise mit einem sogenannten Spike oder einem andersartigen Anschlussmittel gekoppelt oder also solcher ausgebildet, mittels dem eine Fluidkammer des Flüssigkeitsbehälters angezapft werden kann. An einen zweiten Anschluss der 3 - Wege - Weiche (des 3 - Wege - Hahns) ist ein arterieller Leitungsabschnitt und an einen dritten Anschluss der 3 - Wege - Weiche (des 3 - Wege - Hahns) ist ein venöser Leitungsabschnitt anschließbar. Die 3 - Wege - Weiche (vorzugsweise der 3 - Wege - Hahn) ist vorzugsweise manuell in wenigstens drei Stellungen bringbar bzw. schaltbar, von denen in einer ersten Schaltstellung der erste Anschluss ausschließlich mit dem zweiten Anschluss fluidverbunden bzw. eine Fluidströmung zwischen diesen beiden Anschlüssen zugelassen ist und der dritte Anschluss gesperrt ist (single - Pass - Schaltstellung), in einer zweiten Schaltstellung der zweite Anschluss mit dem dritten Anschluss fluidverbunden bzw. eine Fluidströmung zwischen diesen beiden Anschlüssen zugelassen ist und der erste Anschluss gesperrt ist (Rezirkulation - Schaltstellung) und in einer dritten Schaltstellung alle drei Anschlüsse voneinander getrennt bzw. gesperrt sind.

Durch eine derart ausgerüstete Rezirkulationsvorrichtung ist es möglich, den Flüssigkeitsbehälter für den Befüllvorgang an den arteriellen Leitungsabschnitt zu koppeln, indem die 3 - Wege - Weiche, vorzugsweise der 3 - Wege - Hahn in die erste Schaltstellung gebracht wird, und den arteriellen Leitungsabschnitt mit dem venösen Leitungsabschnitt für den Rezirkulationsvorgang zu verbinden, indem die 3 - Wege - Weiche, vorzugsweise der 3 - Wege - Hahn in die zweiten Schaltstellung gebracht wird. Da in dieser zweiten Schaltstellung der erste Anschluss die 3 - Wege - Weiche, vorzugsweise des 3 - Wege - Hahns gesperrt ist, wird der Flüssigkeitsbeutel vom hydraulischen System/Kreis des extrakorporalen Blutbehandlungsgeräts getrennt, sodass die darin sich befindende Restflüssigkeit nicht von der im hydraulischen System rezirkulierenden Flüssigkeit kontaminiert wird. Da in der dritten Schaltstellung der 3 - Wege - Weiche, vorzugsweise des 3 - Wege - Hahns dessen sämtliche Anschlüsse gesperrt sind, können in dieser Schaltstellung die beiden Leitungsabschnitte des hydraulischen Systems abgeklemmt und an den Patienten für eine Behandlung angelegt werden.

Der Flüssigkeitsbehälter kann somit für mehrere aufeinanderfolgende Behandlungen eingesetzt werden in Abhängigkeit von dessen Füllvolumens, sodass keine Flüssigkeit verloren geht. Des Weiteren kann die Rezirkulationsvorrichtung mit einem konventionellen/universellen medizinischen Flüssigkeitsbehälter ausgerüstet sein, der gegenüber den für extrakorporale Blutbehandlungsgeräte speziell angefertigten Behältern mit zwei Anschlüssen preisgünstiger ist. Schließlich braucht der verwendete Flüssigkeitsbehälter gar keinen Anschluss insbesondere für den Fall, dass an die 3 - Wege - Weiche, vorzugsweise den 3 - Wege - Hahn ein sogenannter Spike angeschlossen oder mit diesem zu einer integralen Baueinheit zusammengefasst ist. Im letzteren Fall kann der Spike quasi übergangslos, d.h. ohne Zwischenschaltung eines (überbrückenden) Leitungsstücks unmittelbar mit dem ersten Anschluss vorzugsweise einstückig verbunden sein.

Die Erfindung wird nachstehend anhand eines bevorzugten Ausführungsbeispiels unter Bezugnahme auf die begleitenden Figuren näher erläutert.
Fig. 1 zeigt eine Rezirkulationsvorrichtung eines extrakorporealen Blutbehandlungsgeräts gemäß einem bevorzugten Ausführungsbeispiel der Erfindung,
Fig. 2 zeigt eine geschlossene Schaltstellung eines 3 - Wege - Hahns der Rezirkulationsvorrichtung gemäß Fig. 1 als eine mögliche Variante der erfindungsgemäßen 3 - Wege - Weiche, wobei an dieser Stelle bereits darauf hingewiesen wird, dass beispielsweise auch ein Y- oder T-Stück mit Schlauchsperrmittel (Schlauchklemmen) an jedem Zweigweg vorgesehen sein kann,
Fig. 3 zeigt eine "single-pass" Schaltstellung des 3 - Wege - Hahns der Rezirkulationsvorrichtung gemäß Fig. 1, in der eine Flüssigkeitsquelle (Flüssigkeitsbehälter) ausschließlich mit einem arteriellen Leitungsabschnitt des extrakorporalen Blutbehandlungsgeräts fluidverbunden ist,
Fig. 4 zeigt eine "Rezirkulation" Schaltstellung des 3 - Wege - Hahns der Rezirkulationsvorrichtung gemäß Fig. 1, in welcher der arterielle Leitungsabschnitt ausschließlich mit einem venösen Leitungsabschnitt des extrakorporalen Blutbehandlungsgeräts fluidverbunden (kurzgeschlossen) ist und die Flüssigkeitsquelle vom extrakorporalen Blutbehandlungsgerät fluidgetrennt ist und
Fig. 5 zeigt eine andere geschlossene Schaltstellung eines 3 - Wege - Hahns der Rezirkulationsvorrichtung gemäß Fig. 1,

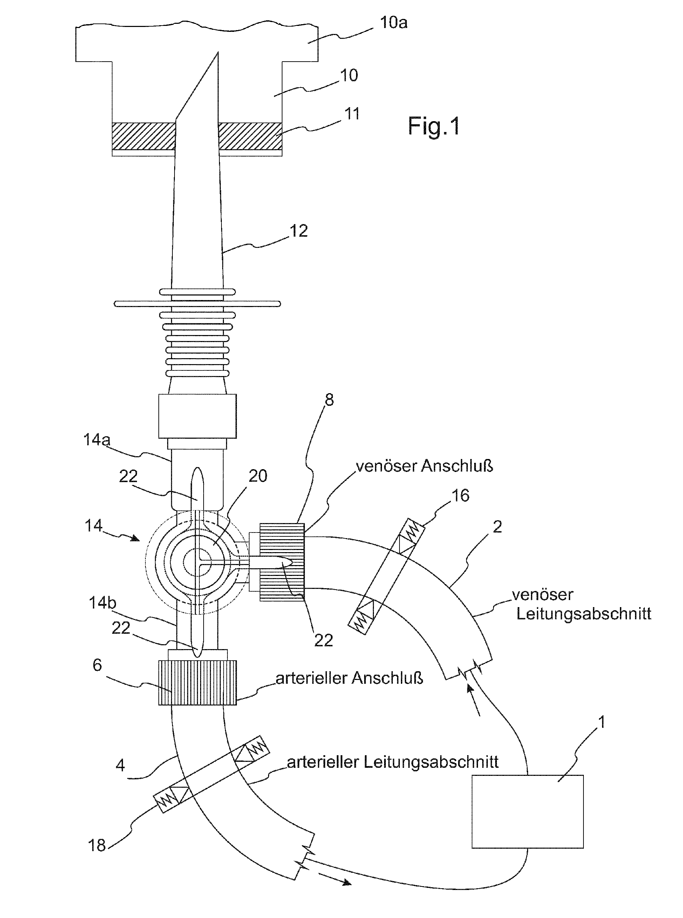

Gemäß der Fig. 1 hat ein extrakorporales Blutbehandlungsgerät 1, beispielsweise eine Dialysemaschine ein internes hydraulisches Leitungssystem (nachfolgend als Fluidleitungssystem bezeichnet), durch das während einer Behandlungsphase auf der Maschinenseite beispielsweise eine Blut - Reinigungsflüsssigkeit (Dialysierflüssigkeit) geleitet wird und auf der Patientenseite Blut extrakorporal hindurchfließt, wobei das maschinenseitige und patientenseitige Fluidleitungssystem im Fall einer Dialysemaschine durch einen nicht weiter dargestellten Dialysator (Filter) fluidisch getrennt sind.

Hierfür hat das Fluidleitungssystem patientenseitig einen venösen sowie einen arteriellen Leitungsabschnitt 2, 4, vorzugsweise jeweils Schlauchabschnitt mit jeweils endseitig angeordneten/ ausgebildeten Anschlüssen 6, 8, an die beispielsweise Injektionsnadeln oder Kanülen (nicht dargestellt) anschließbar sind, welche in einen Patientenkörper einführbar sind.

Um ein Ausschwemmen von ggf. herstellungsbedingten Verunreinigungen insbesondere im patientenseitigen Fluidleitungssystem zu vermeiden, hat das extrakorporale Blutbehandlungsgerät 1 eine Rezirkulationsvorrichtung, mittels welcher das Fluidleitungssystem in der Regel vor jeder Patientenbehandlung gereinigt wird.

Gemäß der Fig. 1 hat die Rezirkulationsvorrichtung gemäß der vorliegenden Erfindung eine Flüssigkeitsquelle vorzugsweise in Form eines universellen Flüssigkeitsbehälters 10 mit einem einzigen Auslass 11, der beim vorliegenden Ausführungsbeispiel mittels eines Spike 12 punktiert wird, um Flüssigkeit aus dem Füssigkeitsbehälter 10 abzuzapfen. Der Aufbau der Spike 12 entspricht dem bekannter Spikekonstruktionen, sodass an dieser Stelle auf dessen Aufbau nicht weiter eingegangen werden muss. Ferner sei darauf hingewiesen, dass beispielsweise im Fall eines Luer-Lock- oder andersartigen Anschlusses auf der Behälterseite der Spike durch ein entsprechendes Anschlussstück auf Seiten der Rezirkulationsvorrichtung ersetzt sein kann.

Des Weiteren hat die erfindungsgemäße Rezirkulationsvorrichtung eine 3 - Wege - Weiche, vorzugsweise einen 3 - Wege - Hahn 14, der dem Spike 12 (Anschlussstück) in Strömungsrichtung weg vom Flüssigkeitsbehälter 10 gesehen, unmittelbar nachgeschaltet ist. Im vorliegenden Fall ist der Spike 12 direkt (ohne Zwischenschaltung eines zusätzlichen Leitungsstücks) an den 3 - Wege - Hahn 14 angeschlossen. Alternativ kann der Spike 12 auch einstückig bzw. als eine Baueinheit mit dem 3 - Wege - Hahn 14 ausgebildet sein.

Der 3 - Wege - Hahn 14 hat hierfür einen ersten Anschluss oder Fluideinlass 14a, der mit dem Spike 12 fluidverbunden ist, bzw. an den die Flüssigkeitsquelle 10 anschließbar/angeschlossen ist. Des Weiteren hat der 3 - Wege - Hahn 14 einen zweiten Anschluss 14b, an den der arterielle Leitungsabschnitt 4 des patientenseitigen Fluidleitungssystems des extrakorporalen Blutbehandlungsgeräts 1 anschließbar ist. Schließlich hat der 3 - Wege - Hahn 14 einen dritten Anschluss 14c, an den der venöse Leitungsabschnitt 2 des patientenseitigen Fluidleitungssystems des extrakorporalen Blutbehandlungsgeräts 1 anschließbar ist. Sowohl der venöse wie auch der arterielle Leitungsabschnitt 2, 4 ist jeweils mit einer Schlauchklemme 16, 18 oder dergleichen Sperrmittel versehen, um den zugehörigen Schlauchabschnitt wahlweise temporär zu verschließen.

Der 3 - Wege - Hahn 14 hat vorliegend einen manuell betätigbaren Drehverschluss bestehend aus einem drehbaren zylindrischen Ventilkolben 20, der stirnseitig mit einer Handhabe vorzugsweise in Form von (drei) Eingriffsflügeln 22 versehen/ausgebildet ist. Der Ventilkolben hat eine zentrale Längsbohrung von der drei in Umfangsrichtung gleichmäßig beabstandete Radialbohrungen abzweigen. Die Eingriffsflügel 22 sind so angeordnet, dass sie sich längs der Radialbohrungen ausrichten und so die Strömungsrichtung der Radialbohrungen anzeigen. Ein solcher 3 - Wege - Hahn ist aus dem Stand der Technik hinlänglich bekannt, sodass an dieser Stelle auf eine weitere Beschreibung insbesondere von dessen Funktion verzichtet werden kann.

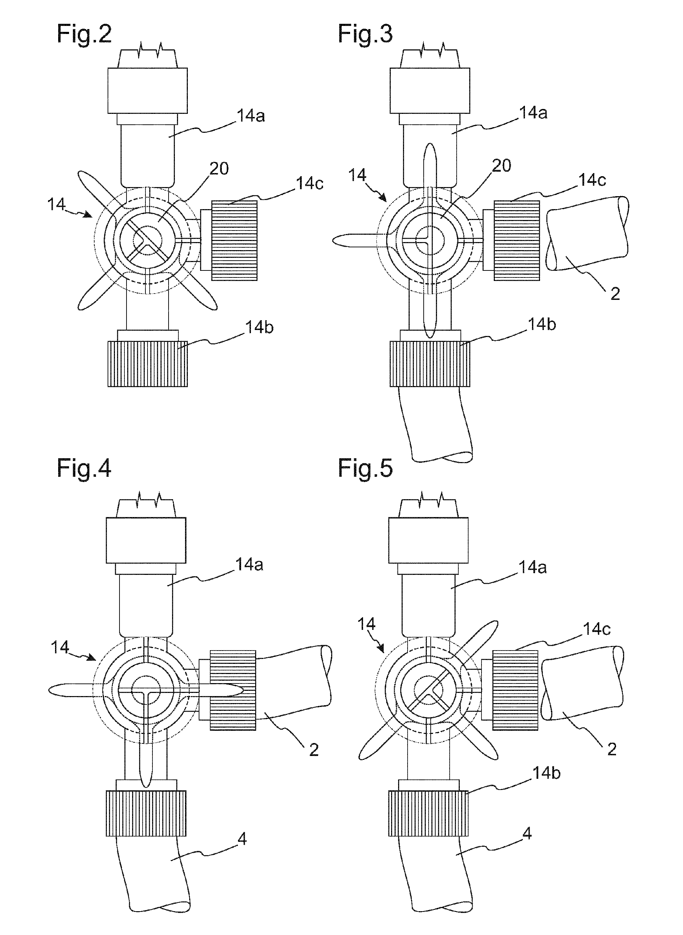

In den Fig. 2 bis 5 sind die erfindungsgemäß beabsichtigten Schaltstellungen des 3 - Wege - Hahn 14 in Abhängigkeit von den aktuellen Betriebsphasen des extrakorporalen Blutbehandlungsgeräts 1 dargestellt, welche nachfolgend in Zusammenhang mit den dadurch bezweckten Funktionen beschrieben werden. Gemäß der Fig. 2 ist der 3 - Wege - Hahn 14 in einer Sperrposition gezeigt, in der alle drei Anschlüsse 14a - 14c geschlossen sind. In dieser Schaltstellung kann der Spike 12 den Auslass 12 des Universal - Flüssigkeitsbehälters 10 punktieren und somit die darin ( in der vom Behälter ausgebildeten Kammer 10a ) gespeicherte Flüssigkeit anzapfen, ohne dass Flüssigkeit zur Atmosphäre hin verloren geht.

Die Fig. 3 zeigt die so genannte "single pass" Schaltstellung, in welcher der erste Anschluss 14a mit dem zweiten Anschluss 14b fluidverbunden ist, wohingegen der dritte Anschluss 14c gesperrt wird. In dieser Schaltstellung ist bereits der arterielle Leitungsabschnitt 4 an den zweiten Anschluss 14b angeschlossen, wobei jedoch der venöse Leitungsabschnitt 2 zur Atmosphäre hin offen ist oder an einen Abfluss/Sammelbehälter angeschlossen ist.

In dieser Schaltposition wird Flüssigkeit (NaCl-Lösung) über den 3 - Wege - Hahn 14 in den arteriellen Leitungsabschnitt 4 geleitet und dadurch das patientenseitige Fluidleitungssystem fortlaufend geflutet, solange, bis die Flüssigkeit aus dem venösen Leitungsabschnitt 2 heraus läuft. D.h. während dieses System-Füllvorgangs dient der venöse Leitungsabschnitt 2 als Entlüftung. Dabei sei darauf hingewiesen, dass während dieses Vorgangs die Schlauchklemmen 16, 18 natürlich geöffnet sind.

Sobald das System mit Flüssigkeit aus dem Flüssigkeitsbehälter 10 aufgefüllt ist, wird der venöse Leitungsabschnitt an den dritten Anschluss 14c des 3 - Wege - Hahns 14 angeschlossen und der 3 - Wege - Hahn 14 in die Schaltstellung gemäß der Fig. 4 verstellt, welche als "Rezirkulation" Schaltstellung bezeichnet werden kann. In dieser Schaltstellung sind der zweite und der dritte Anschluss 14b, 14c des 3 - Wege - Hahns 14 miteinander fluidverbunden, wohingegen der erste Anschluss 14a gesperrt wird.

Wird nunmehr die im Fluidleitungssystem befindliche Flüssigkeit zirkuliert, durchströmt diese ausgehend vom venösen Leitungsabschnitt 2 den 3 - Wege - Hahn 14 und wird von dort wieder in den arteriellen Leitungsabschnitt 4 weiter geleitet, ohne dass Flüssigkeit in den Flüssigkeitsbehälter 10 eindringen kann. Die darin gespeicherte Flüssigkeit bleibt somit unkontaminiert.

Nach einer vorbestimmten Rezirkulationsdauer wird der 3 - Wege - Hahn 14 in die in Fig. 5 gezeigte Schaltstellung weitergeschaltet, in welcher erneut alle drei Anschlüsse 14a - 14c gesperrt sind. Es ist zu erkennen, dass sich die Schaltstellung gemäß der Fig. 5 von der Schaltstellung gemäß der Fig. 2 unterscheidet, da der Ventilkolben 20 nicht einfach in die erste Sperrstellung gemäß Fig. 2 voll - rückgedreht wurde sondern in die zweite Sperrstellung gemäß der Fig. 5 teil - rückgedreht wurde, welche demnach der ersten Sperrstellung diametral gegenüberliegt. Wäre der Ventilkolben 20 nämlich voll - rückgedreht worden, hätte er zumindest die "Single pass" Schaltstellung temporär durchlaufen, in der kontaminierte Flüssigkeit aus dem Fluidleitungssystem in den Flüssigkeitsbehälter hätte eindringen können.

Sobald der 3 - Wege - Hahn 14 gesperrt ist, wird nunmehr der venöse Leitungsabschnitt 2 erneut vom 3 - Wege - Hahn 14 abgeklemmt und der Ventilkolben 20 in die "single - pass" Stellung gemäß Fig. 3 gedreht, um die kontaminierte Flüssigkeit aus dem Fluidleitungssystem zu spülen. Mit Beendigung dieses Vorgangs ist der Befüll-/Rezirkulationszyklus abgeschlossen.

Abschließend wird der 3 - Wege - Hahn 14 erneut gesperrt, alle Schlauchklemmen 16, 18 in Sperrstellung gebracht und auch der arterielle Leitungsabschnitt vom 3 - Wege - Hahn 14 abgeklemmt, um zusammen mit dem venösen Leitungsabschnitt am Patientenkörper angelegt werden zu können.

Die vorliegende Erfindung betrifft zusammenfassend eine Rezirkulationsvorrichtung eines extrakorporalen Blutbehandlungsgeräts 1 mit einem vorzugsweise universellen medizinischen Flüssigkeitsbehälter 10, an den wahlweise ein arterieller Leitungsabschnitt 4 eines Fluidleitungssystems des extrakorporalen Blutbehandlungsgeräts 1 anschließbar ist. Die Rezirkulationsvorrichtung hat ferner eine 3 - Wege - Weiche, vorzugsweise einen 3 - Wege - Hahn 14, der dem universellen medizinischen Flüssigkeitsbehälter 10 unmittelbar nachgeordnet ist. Ein Weg 14a der 3 - Wege - Weiche ist vorzugsweise mit einem Spike 12 oder dergleichen Anschlussmittel gekoppelt oder einstückig mit diesem ausgebildet.

## Patentansprüche

1. Rezirkulationsvorrichtung eines extrakorporalen Blutbehandlungsgeräts (1) mit einem medizinischen Flüssigkeitsbehälter (10), an den wahlweise ein arterieller Leitungsabschnitt (4) eines Fluidleitungs - Systems des extrakorporalen Blutbehandlungsgeräts (1) anschließbar ist, und einer 3 - Wege - Weiche, vorzugsweise einem 3 - Wege - Hahn (14), welche dem medizinischen Flüssigkeitsbehälter (10) unmittelbar nachgeordnet ist,
wobei ein erster Weg (14a) der 3 - Wege - Weiche mit einem Anschlussmittel (12) gekoppelt oder also solches ausgebildet ist, mittels dem eine Fluidkammer (10a) des Flüssigkeitsbehälters (10) anzapfbar ist oder mit dieser fluidverbindbar ist, wobei ein zweiter Weg (14b) der 3 - Wege - Weiche zum Anschließen des arteriellen Leitungsabschnitts (4) und ein dritter Weg (14c) der 3 - Wege - Weiche zum Anschließen eines venösen Leitungsabschnitts (2) des Fluidleitungssystems des extrakorporalen Blutbehandlungsgeräts (1) vorgesehen sind und
wobei die 3 - Wege - Weiche in wenigstens drei Stellungen bringbar oder schaltbar ist, von denen in einer ersten Schaltstellung der erste Weg (14a) mit dem zweiten Weg (14b) fluidverbunden ist oder dazwischen eine Fluidströmung zulässt, und der dritte Weg (14c) gesperrt ist, in einer zweiten Schaltstellung der zweite Weg (14b) mit dem dritten Weg (14c) fluidverbunden oder eine Fluidströmung dazwischen zulässt und der erste Weg (14a) gesperrt ist und in einer dritten Schaltstellung alle drei Wege (14a-14c) gesperrt sind, **dadurch gekennzeichnet, dass**
der Flüssigkeitsbehälter (10) für den Befüllvorgang an den arteriellen Leitungsabschnitt (4) gekoppelt ist, indem die 3 - Wege - Weiche in die erste Schaltstellung gebracht wird, und der arterielle Leitungsabschnitt (4) mit dem venösen Leitungsabschnitt (2) für den Rezirkulationsvorgang verbunden ist, indem die 3 - Wege - Weiche in die zweiten Schaltstellung gebracht wird, wobei in dieser zweiten Schaltstellung der erste Weg (14a) der 3 - Wege - Weiche gesperrt ist und damit der Flüssigkeitsbehälter (10) vom Fluidleitungssystem des extrakorporalen Blutbehandlungsgeräts (10) fluidgetrennt ist.

2. Rezirkulationsvorrichtung nach Anspruch 1, **dadurch gekennzeichnet, dass** der 3 - Wege - Hahn (14) manuell betätigbar ist, wofür dieser einen mit einer Handhabe (22) ausgebildeten Drehkolben (20) aufweist.

3. Rezirkulationsvorrichtung nach Anspruch 1 oder 2, **dadurch gekennzeichnet, dass** in der dritten Schaltstellung die 3 - Wege - Weiche, vorzugsweise des 3 - Wege - Hahns (14) dessen sämtliche Wege (14a - 14c) gesperrt sind, sodass in dieser Schaltstellung die beiden Leitungsabschnitte (2, 4) des Fluidleitungssystems von dem Flüssigkeitsbehälter (10) abklemmbar sind.

4. Rezirkulationsvorrichtung nach einem der vorstehenden Ansprüche, **dadurch gekennzeichnet, dass** an den ersten Weg (14a) der 3 - Wege - Weiche, vorzugsweise des 3 - Wege - Hahns (14) ein Spike als Anschlussmittel (12) angeschlossen oder mit diesem zu einer integralen Baueinheit zusammengefasst ist, der zum Anschließen an den Flüssigkeitsbehälter (10) angepasst ist.

5. Rezirkulationsvorrichtung nach Anspruch 4, **dadurch gekennzeichnet, dass** der Spike (12) übergangslos ohne Zwischenschaltung eines überbrückenden Leitungsstücks unmittelbar mit dem ersten Weg (14a) der 3 - Wege - Weiche, vorzugsweise des 3 - Wege - Hahns (14) vorzugsweise einstückig verbunden ist.

6. Rezirkulationsvorrichtung nach einem der vorstehenden Ansprüche, **dadurch gekennzeichnet, dass** der Flüsigkeitsbehälter (10) als ein Universalbehälter mit ausschließlich einem Flüssigkeitsauslass oder einer einzigen Anzapfstelle (11) für einen Spike (12) oder einem entsprechenden Anschlussmittel ausgebildet ist.

7. Extrakorporales Blutbehandlungsgerät mit einem internen Fluidleitungssystem, das einen arteriellen und einen venösen Leitungsabschnitt (2, 4) für ein Anschließen des Fluidleitungssystems an einen Patienten hat, **gekennzeichnet durch** eine Rezirkulationsvorrichtung gemäß einem der Ansprüche 1 bis 6

8. Verwendung einer Rezirkulationsvorrichtung gemäß einem der Ansprüche 1 bis 6 in einer Dialysemaschine (1), welche ein patientenseitiges Fluidleitungssystem mit einem arteriellen sowie einem venösen Leitungsabschnitt (2, 4) hat, die wahlweise mit der Rezirkulationsvorrichtung zu deren Befüllung fluidverbindbar sind.

9. Verfahren zur Durchführung eines Befüll- und Rezirkulationsvorgangs eines blutseitigen Leistungssystems einer Dialysemaschine unter Verwendung einer Rezirkulationsvorrichtung nach einem der Ansprüche 1 bis 6 mit den Schritten:
a. Anschließen des Spike 3-Wege-Hahns (14) an den einen Fluidanschluss des medizinischen Fluidbehälters (10) bei vollständig gesperrtem 3-Wege-Hahn (14) sowie Anschließen des arteriellen Leitungsabschnitts (4) an den zweiten Weg (14b) des noch gesperrten-3-Wege-Hahn (14) bei zur Umgebung hin geöffnetem venösem Fluidleitungsabschnitt (2),
b. Durchspülen eines Fluidströmungspfads entlang des arteriellen Fluidleitungsabschnitts (4) des extrakorporalen Blutbehandlungsgeräts (1) und des venösen Fluidleitungsabschnitts (2) durch Fluidverbinden des Fluidbehälters (10) mit dem zweiten Weg (14b) am 3-Wege-Hahn (14),
c. vollständiges Sperren des 3-Wege-Hahns (14) und Anschließen des venösen Fluidleitungsabschnitts (2) an den dritten Weg (14c) des 3-Wege-Hahns,
d. Rezirkulieren des eingefüllten Fluids in dem Fluidströmungspfad durch Kurzschließen des zweiten und dritten Wegs (14b, 14c) am 3-Wege-Hahn bei gleichzeitigem Sperren des Fluidbehälters (12),
e. vollständiges Sperren des 3-Wege-Hahns (14) und Abkoppeln des venösen Fluidleitungsabschnitts (2) vom dritten Weg (14c) sowie erneutes Durchspülen gemäß Schritt b., und
f. vollständiges Sperren des 3-Wege-Hahns als Vorbereitung für das darauffolgende Anschließen der befüllten arteriellen und venösen Fluidleitungsabschnitte (2, 4) an einen Patienten.

## Claims

1. Recirculation apparatus of an extracorporeal blood treatment device (1) having a medical fluid container (10) to which optionally an arterial conducting portion (4) of a fluid conduction system of the extracorporeal blood treatment device (1) can be connected, and a 3-way switch, preferably a 3-way tap (14), which is connected directly downstream to the fluid source,
wherein a first path (14a) of the 3-way switch is coupled with a connecting means (12) or is configured as such, by means of which a fluid chamber (10a) of the fluid container (10) can be tapped or is fluidly connectible with this, wherein a second path (14b) of the 3-way switch for connecting the arterial conducting portion (4) is provided and a third path (14c) of the 3-way switch for connecting a venous conducting portion (2) of the fluid conduction system of the extracorporeal blood treatment device (1) is provided,
wherein the 3-way switch can be brought into or switched to at least three positions, from which, in a first switching position, the first path (14a) is fluidly connected with the second path (14b) or allows a fluid flow between them, and the third path (14c) is blocked; in a second switching position, the second path (14b) is fluidly connected with the third path (14c) or allows a fluid flow between them, and the first path (14a) is blocked; and in a third switching position, all three paths (14a-14c) are blocked, **characterized in that**
the liquid container (10) is coupled to the arterial conducting portion (4) for the filling process by bringing the 3-way switch to the first switching position, and the arterial conducting portion (4) is connected to the venous conducting portion (2) for the recirculation process by bringing the 3-way switch to the second switching position, wherein, in this second switching position, the first path (14a) of the 3-way switch is blocked and the fluid container (10) is thereby fluidly separated from the fluid conduction system of the extracorporeal blood treatment device (10).

2. Recirculation apparatus according to claim 1, **characterized in that** the 3-way tap (14) is manually operable, for which said tap comprises a rotary piston (20) configured with a handle (22).

3. Recirculation apparatus according to claim 1 or 2, **characterized in that** in the third switching position of the 3-way switch, preferably of the 3-way tap (14), all of its paths (14a - 14c) are blocked, such that in this switching position, both of the conducting portions (2, 4) of the fluid conduction system are disconnectable from the fluid container (10).

4. Recirculation apparatus according to one of the above claims, **characterized in that** a spike as connecting means (12) is connected to the first path (14a) of the 3-way switch, preferably of the 3-way tap (14), or is combined with this to form an integral unit, wherein the spike is suited for being connected to the fluid container (10).

5. Recirculation apparatus according to claim 4, **characterized in that** the spike (12) is connected, preferably integrally connected, transition-free and without interconnection of a bridging conducting piece, directly with the first path (14a) of the 3-way switch, preferably of the 3-way tap (14).

6. Recirculation apparatus according to one of the above claims, **characterized in that** the fluid container (10) is configured as a universal container having exclusively one fluid outlet or a single tapping point (11) for a spike (12) or a corresponding connecting means.

7. Extracorporeal blood treatment device having an internal fluid conduction system that has an arterial and a venous conducting portion (2, 4) for a connection of the fluid conduction system to a patient, **characterized by** a recirculation apparatus according to any one of claims 1 to 6.

8. Application of a recirculation apparatus according to any one of claims 1 to 6 in a dialysis machine (1) which has a fluid conduction system on the patient side having an arterial as well as a venous conducting portion (2, 4) which are optionally fluidly connectible to the recirculation apparatus.

9. Method for performing a recirculation procedure of a blood-side conduction system of a dialysis machine by using a recirculation apparatus according to any one of claims 1 to 6, comprising the steps of:
a. connecting the spike 3-way tap (14) to the fluid connection of the medical fluid container (10) when the 3-way tap (14) is completely blocked as well as connecting the arterial conducting portion (4) to the second path (14b) of the still blocked 3-way tap (14) when the venous fluid conducting portion (2) is opened to the surroundings,
b. flushing a fluid flow path along an arterial fluid conducting portion (4) of the extracorporeal blood treatment device (1) and of the venous fluid conducting portion (2) by fluid connecting the fluid container (10) with the second path (14b) at the 3-way tap (14),
c. completely locking the 3-way tap (14) and connecting the venous fluid conducting portion (2) to the third path (14c) of the 3-way tap,
d. recirculating the poured-in fluid in the fluid flow path by directly connecting the second and the third path (14b, 14c) at the 3-way tap while simultaneously blocking the fluid container (12),
e. completely blocking the 3-way tap (14) and decoupling the venous fluid conducting portion (2) from the third way (14c) as well as repeated flushing according to step b., and
f. completely blocking the 3-way tap as preparation for the subsequent connecting of the filled arterial and venous fluid conducting portions (2, 4) to the patient.

## Revendications

1. Dispositif de recirculation d'un appareil de traitement sanguin extracorporel (1) comprenant un contenant de liquide (10) médical, auquel au choix un tronçon de conduite artérielle (4) d'un système de conduite de fluide de l'appareil de traitement sanguin extracorporel (1) peut être raccordé, et un système d'aiguillage à 3 voies, de préférence un robinet à 3 voies (14), qui est disposé directement en aval du contenant de liquide (10) médical,
une première voie (14a) du système d'aiguillage à 3 voies étant couplée à un moyen de raccordement (12) ou étant réalisée en tant que tel, lequel moyen de raccordement permet à une chambre de fluide (10a) du contenant de liquide (10) de s'approvisionner et qui peut être relié de manière fluidique à cette dernière,
une deuxième voie (14b) du système d'aiguillage à 3 voies étant prévue aux fins du raccordement du tronçon de conduite artérielle (14b) et une troisième voie (14c) du système d'aiguillage à 3 voies étant prévue aux fins du raccordement d'un tronçon de conduite veineuse (2) du système de conduite de fluide de l'appareil de traitement sanguin extracorporel (1), et
le système d'aiguillage à 3 voies pouvant être amené ou commuté dans au moins trois positions, la première voie (14a) étant en liaison fluidique avec la deuxième voie (14b) dans une première position de commutation ou autorisant entre elles un écoulement de fluide,
et la troisième voie (14c) étant fermée, la deuxième voie (14b) étant en liaison fluidique avec la troisième voie (14c) dans une deuxième position de commutation ou autorisant entre elles un écoulement de fluide et la première voie (14a) étant fermée et les trois voies (14a - 14c) étant fermées dans une troisième position de commutation, **caractérisé en ce que**
le contenant de liquide (10) est couplé au tronçon de conduite artérielle (4) en vue de l'opération de remplissage, tandis que le système d'aiguillage à 3 voies est amené dans la première position de commutation, et **en ce que** le tronçon de conduite artérielle (4) est relié au tronçon de conduite veineuse (2) en vue de l'opération de recirculation, tandis que le système d'aiguillage à 3 voies est amené dans la deuxième position de commutation, la première voie (14a) du système d'aiguillage à 3 voies étant fermée dans ladite deuxième position de commutation et le contenant de fluide (10) étant de ce fait séparé fluidiquement du système de conduite de fluide de l'appareil de traitement sanguin extracorporel (1)

2. Dispositif de recirculation selon la revendication 1, **caractérisé en ce que** le robinet à 3 voies (14) peut être actionné manuellement, ce dernier présentant à cet effet un piston rotatif (20) réalisé avec une poignée (22).

3. Dispositif de recirculation selon la revendication 1 ou 2, **caractérisé en ce que** dans la troisième position de commutation du système d'aiguillage à 3 voies, de préférence du robinet à 3 voies (14), toutes les voies (14a - 14c) de ce dernier sont fermées de sorte que dans ladite position de commutation, les deux tronçons de conduite (2, 4) du système de conduite de fluide peuvent être détachés du contenant de fluide (10).

4. Dispositif de recirculation selon l'une quelconque des revendications précédentes, **caractérisé en ce qu'**une pointe est raccordée en tant que moyen de raccordement (12) à la première voie (14a) du système d'aiguillage à 3 voies, de préférence du robinet à 3 voies (14) ou est assemblée à ce dernier pour former une unité modulaire intégrale, laquelle pointe est adaptée aux fins du raccordement au contenant de fluide (10).

5. Dispositif de recirculation selon la revendication 4, **caractérisé en ce que** la pointe (12) est reliée de préférence d'un seul tenant, sans transition, sans intercaler une partie de conduite de pontage, directement à la première voie (14a) du système d'aiguille à 3 voies, de préférence du robinet à 3 voies (14).

6. Dispositif de recirculation selon l'une quelconque des revendications précédentes, **caractérisé en ce que** le contenant de liquide (10) est réalisé sous la forme d'un contenant universel comprenant exclusivement une évacuation de liquide ou un unique point d'approvisionnement (11) pour une pointe (2) ou un moyen de raccordement correspondant.

7. Appareil de traitement sanguin extracorporel comprenant un système de conduite de fluide interne, qui comprend un tronçon de conduite artérielle et un tronçon de conduite veineuse (2, 4) en vue d'un raccordement du système de conduite de fluide à un patient, **caractérisé par** un dispositif de recirculation selon l'une quelconque des revendications 1 à 6.

8. Utilisation d'un dispositif de recirculation selon l'une quelconque des revendications 1 à 6,
dans une machine de dialyse (1), qui est équipée d'un système de conduite de fluide côté patient comprenant un tronçon de conduite artérielle et un tronçon de conduite veineuse (2, 4), qui peuvent être reliés de manière fluidique au choix au dispositif de recirculation aux fins du remplissage de ce dernier.

9. Procédé servant à exécuter une opération de remplissage et de recirculation d'un système de soin côté sang d'une machine de dialyse en utilisant un dispositif de recirculation selon l'une quelconque des revendications 1 à 6, comprenant les étapes suivantes consistant à :
a. raccorder la pointe du robinet à 3 voies (14) à l'un des raccords de fluide du contenant de fluide médical (10) lorsque le robinet à 3 voies (14) est intégralement fermé et raccorder le tronçon de conduite artérielle (4) à la deuxième voie (14b) du robinet à 3 voies (14) encore fermé lorsque le tronçon de conduite de fluide (2) veineuse est ouvert en direction de l'environnement ;
b. rincer un trajet d'écoulement de fluide le long du tronçon de conduite de fluide artériel (4) de l'appareil de traitement sanguin extracorporel (1) et du tronçon de conduite de fluide veineux (2) en reliant de manière fluidique le contenant de fluide (10) à la deuxième voie (14b) au niveau du robinet à 3 voies (14) ;
c. fermer intégralement le robinet à 3 voies (14) et raccorder le tronçon de conduite de fluide veineux (2) à la troisième voie (14c) du robinet à 3 voies ;
d. faire recirculer le fluide transvasé dans le chemin d'écoulement de fluide en court-circuitant la deuxième et la troisième voie (14b, 14c) au niveau du robinet à 3 voies tout en fermant de manière simultanée le contenant de fluide (12) ;
e. fermer intégralement le robinet à 3 voies (14) et découpler le tronçon de conduite de fluide veineux (2) de la troisième voie (14c) et renouveler le rinçage selon l'étape b., et
f. fermer intégralement le robinet à 3 voies comme préparation en vue du raccordement qui suit des sections de conduites de fluide artérielle et veineuse (2, 4) remplies à un patient.
